Europäisches Patentamt

European Patent Office

Office européen des brevets

(19)

(11) Veröffentlichungsnummer: **0 257 355**
**A2**

(12) # EUROPÄISCHE PATENTANMELDUNG

(21) Anmeldenummer: **87111154.8**

(22) Anmeldetag: **01.08.87**

(51) Int. Cl.⁴: **A61M 16/00 , G01D 3/00**

(30) Priorität: **27.08.86 DE 3629126**

(43) Veröffentlichungstag der Anmeldung:
**02.03.88 Patentblatt 88/09**

(84) Benannte Vertragsstaaten:
**DE FR GB NL SE**

(71) Anmelder: **Drägerwerk Aktiengesellschaft**
**Moislinger Allee 53-55**
**D-2400 Lübeck 1(DE)**

(72) Erfinder: **Heitmann, Hans Heinrich, Dr.**
**Dipl.-Phys.**
**Clever Landstrasse 25g**
**D-2406 Stockelsdorf(DE)**
Erfinder: **Beissert, Wolfgang**
**Parkweg 9b**
**D-2406 Stockelsdorf(DE)**

(54) **Drücküberwachungsvorrichtung für eine Atmungsvorrichtung.**

(57) Eine Drucküberwachungsvorrichtung für eine Atmungsvorrichtung, welche ein druckempfindliches Fühlerelement besitzt, durch das sowohl beim Überschreiten als auch beim Unterschreiten einer mit einem Grenzwertgeber vorgebbaren Druckschwelle über ein durch einen Zeitgeber bestimmbares Zeitintervall hinaus ein Signalgeber tätigbar ist, soll derart verbessert werden, daß eine Überwachung von Diskonnektion und/ oder Stenose in den atemgasführenden Leitungen der Atmungsvorrichtung auch bei sehr geringen Atemwegsdruckschwankungen, für deren Überwachung die bekannte Vorrichtung zu unempfindlich ist, und auch bei leicht schwankendem Verlauf von Atemwegsdruckmaxima und -minima um eine Druckmittellinie herum möglich ist. Dazu ist vorgesehen, daß dem Zeitgeber (5) ein Komparator (1) zur Aktivierung des Signalgebers (6) jeweils bei Überschreiten einer oberen Druckschwelle und bei Unterschreiten einer unteren Druckschwelle vorgeschaltet ist.

## Drucküberwachungsvorrichtung für eine Atmungsvorrichtung

Die Erfindung betrifft eine Drucküberwachungsvorrichtung für eine Atmungsvorrichtung, welche ein druckempfindliches Fühlerelement besitzt, durch das sowohl beim Überschreiten als auch beim Unterschreiten einer mit einem Grenzwertkomparator vorgegebenen Druckschwelle über ein durch einen Zeitgeber bestimmbares Zeitintervall hinaus ein Signalgeber betätigbar ist.

Eine derartige Drucküberwachungsvorrichtung ist aus der DE-AS 12 78 704 bekannt geworden.

Diese Überwachungsvorrichtung besitzt eine Prüfkammer, deren eine Wand durch eine vorgespannte Membran als druckempfindliches Fühlerelement gebildet ist. Diese Prüfkammer ist über eine Druckleitung mit dem Luftweg zwischen dem Patienten und einem Beatmungsgerät verbunden. An die Membran ist über eine Betätigungsstange ein Umschalter als Grenzwertkomparator angeschlossen. Der Umschalter - schaltet bei jedem Atemzyklus wechselweise zwei Zeitgebereinheiten, sobald der Druck in der Prüfkammer die durch die vorgespannte Membran festgelegte Druckschwelle über-bzw. unterschreitet. Beim Umlegen des Umschalters wird die jeweils geschaltete Zeitgebereinheit in Gang gesetzt und die andere abgeschaltete Zeitgebereinheit in ihne Ruhestellung zurückgestellt. Wenn innerhalb der Prüfkammer der eingestellte Druck über die jeweiligen vorgegebenen Verzögerungszeitintervalle der Zeitgebereinheiten hinaus nicht erreicht bzw. überschritten wird, wird ein Signalstromkreis geschlossen und damit ein Warnsignal ausgelöst. Die Verzögerungszeitintervalle bis zum Auftreten des Signals verhindern eine sofortige Schaltung beim Über-bzw. Unterschreiten der eingestellten Druckschwelle, wie es sich im Wechsel der Atemphasen einstellt. Ein Alarmsignal erfolgt nur nach einer Störung des normalen Ablaufs der vom Beatmungsgerät abgegebenen Beatmungszyklen über das vorgegebene Zeitintervall hinaus. Dies kann insbesondere durch Undichtigkeiten (Diskonnektion) oder durch Verunreinigungen bzw. Verstopfungen der atemgasführenden Leitungen (Stenose) im Beatmungsgerät oder in den Zuführungsleitungen zum Patienten erfolgen.

Bei der Anwendung der bekannten Drucküberwachungsvorrichtung erweist es sich als nachteilig, daß der Druckverlauf innerhalb der Prüfkammer während eines maschinellen Atemzyklus den eingestellten Druck zumindest erreichen bzw. sogar durchschreiten muß, damit der Umschalter betätigt wird und so ein wechselseitiges Rückstellen und Aktivieren der beiden Zeitgeber erfolgt. Es ist daher erforderlich, daß der Druckverlauf innerhalb der Prüfkammer den eingestellten Überwachungsdruck durchschreitet. Nur unter dieser Bedingung ist eine korrekte und eindeutige Alarmgabe gewährleistet.

Im täglichen Betrieb eines Beatmungsgerätes ist jedoch die Form der Druckkurve bei unterschiedlichen Beatmungsarten und bei gelegentlich erwünschten Spontanatemzügen nicht in der Weise gleichbleibend, daß ein gleichbleibendes periodisches Durchlaufen der Warngrenze innerhalb der vorgegebenen Verzögerungszeitintervalle vorausgesetzt werden kann. Dadurch kann es vorkommen, daß die periodischen Druckschwankungen die vorgegebene Druckgrenze über das vom Zeitgeber vorgegebene Zeitintervall nicht erreicht, und somit ein Fehlalarm ausgelöst wird. Darüberhinaus erweist es sich als besonders schwierig, die für die Überwachung notwendige Druckschwelle dann einzustellen, wenn die periodischen Druckschwankungen sehr gering werden. Dies ist zum Beispiel dann der Fall, wenn Patienten mit sehr kleinem Atemzugvolumen, wie es zum Beispiel bei Kindern oder Kleinkindern der Fall ist, beatmet und überwacht werden sollen. Die Druckschwankungen können dann unter Umständen nicht mehr ausreichen, um eine genügende Membrankraft zu erzeugen, welche den Umschalter betätigen muß. Insbesondere wenn das Beatmungsgerät einen kontinuierlichen Überdruck zur Beatmung liefert (CPAP), dem der gering schwankende Beatmungsdruckverlauf überlagert ist, reicht die Empfindlichkeit der Druckschalter nicht immer aus, die vorgegebene Druckschwelle zu durchschreiten. Es kommt auch hierbei zu unerwünschten Fehlwarnungen.

Der vorliegenden Erfindung liegt somit die Aufgabe zugrunde, eine Drucküberwachungsvorrichtung der genannten Art derart zu verbessern, daß eine Überwachung von Diskonnektion und/oder Stenose in den atemgasführenden Leitungen der Atmungsvorrichtung auch bei sehr geringen Atemwegsdruckschwankungen, für deren Überwachung die bekannte Vorrichtung zu unempfindlich ist, und auch bei leicht veränderlichem Verlauf von Atemwegsdruckmaxima und -minima um eine Druckmittellinie herum möglich ist.

Die Aufgabe wird dadurch gelöst, daß der Grenzwertkomparator als Fensterkomparator für einen oberen und einen unteren Schwellwert zur Aktivierung des Zeitgebers jeweils bei Überschreiten des oberen Schwellwertes und bei Unterschreiten des unteren Schwellwertes ausgebildet ist.

Durch die Vorgabe von zwei getrennten Druckschwellen, die jeweils für sich über-bzw. unterschritten werden müssen, werden Schwankungen im Atemwegsdruckverlauf, welche innerhalb dieser beiden Grenzlinien bleiben, nicht zur Überwachung des Druckverlaufes herangezogen. Zulässige Variationen der Atemwegsdruckmaxima bzw. -minima, welche innerhalb des so festgelegten erlaubten Schwankungsbereichs bleiben, führen nicht zur Aktivierung des Zeitgebers für eine Alarmabgabe. Auch werden Atemwegsdruckschwankungen nur sehr geringen Ausmaßes innerhalb des Druckfensters, insbesondere beim CPAP-Betrieb, nicht zur Auslösung eines Warnsignales herangezogen. In beiden Fällen ist jedoch nach wie vor eine zuverlässige Überwachung von Diskonnektion und/oder Stenose in den atemgasführenden Leitungen des Beatmungsgerätes gegeben. Diese erfolgt jeweils nur dann, wenn die Atemgasdruckkurve die Grenzen des vorgebbaren Druckfensters überschreitet.

In besonders vorteilhafter Weise kann durch den Fensterkomparator eine feste Fensterbreite vorgebbar sein, deren Lage durch einen Sollwertgeber wählbar ist. Der Sollwertgeber kann dabei entweder einen der beiden Schwellwerte oder zum Beispiel einen Mittelwert bestimmen, wodurch der jeweils andere Schwellwert bzw. die um den Mittelwert gelegenen oberer und unterer Schwellwert durch die Fensterbreite vorgegeben sind.

Um eine separate Einstellung beider Schwellwerte zu ermöglichen, ist in einer weiteren zweckmäßigen Ausgestaltung der Erfindung vorgesehen, den Komparator mit zwei einstellbaren Sollwertgebern zu verbinden. Dadurch wird eine bessere Anpassung an unterschiedliche Druckverhältnisse bei verschiedenen Beatmungsarten und daraus resultierenden Atemwegsdruckverhältnissen erreicht.

Ein Ausführungsbeispiel der Erfindung wird anhand der schematischen Zeichnung dargestellt und im folgenden näher erläutert.

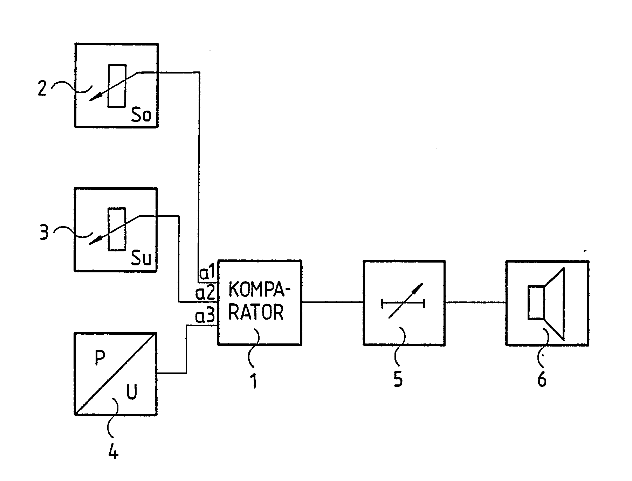

In der einzigen Figur ist ein Blockdiagramm für eine Drucküberwachungsvorrichtung angegeben, die aus einem Komparator (1) besteht, an dessen Eingängen (a1, a2, a3) jeweils ein Sollwertgeber (2) zur Einstellung des oberen Schwellwertes (So) und ein Sollwertgeber (3) für die Einstellung des unteren Schwellwertes (Su) sowie ein Druckspannungswandler (4) als druckempfindliches Element angeschlossen sind. Solange das vom Druck-Spannungswandler (4) abgegebene Signal innerhalb des durch die beiden Sollwertgeber (2, 3) festgelegten Fensters liegt, wird durch ein Signal ein mit dem Ausgang des Komparators (1) verbundener Zeitgeber (5) zurückgesetzt. Sobald das Signal des Druck-Spannungswandlers außerhalb des so festgelegten Druckfensters liegt, wird der Zeitgeber (5) wegen dr· fehlenden Rücksetzimpulse in Gang gesetzt. Erreicht das Signal das Druckfenster, bevor die am Zeitgeber (5) einstellbare Zeit abgelaufen ist, wird er wiederum zurückgesetzt. Erst wenn nach Ablauf des Zeitgebers (5) das Signal immer noch außerhalb des Druckfenster liegt, oder erst danach das Druckfenster erreicht, wird ein an den Zeitgeber angeschlossener Signalgeber (6) aktiviert.

Zur Verwirklichung der beispielhaft angegebenen Drucküberwachungsvorrichtung können bekannte, handelsübliche Baukomponenten verwendet werden.

**Ansprüche**

1. Drucküberwachungsvorrichtung für eine Atmungsvorrichtung, welche ein druckempfindliches Fühlerelement besitzt, durch das sowohl beim Überschreiten als auch beim Unterschreiten einer an einem Grenzwertkomparator vorgegebenen Druckschwelle über ein durch einen Zeitgeber bestimmbares Zeitintervall hinaus ein Signalgeber betätigbar ist, dadurch gekennzeichnet, daß der Grenzwertkomparator als Fensterkomparator (1) für einen oberen (So) und einen unteren (Su) Schwellwert zur Aktivierung des Zeitgebers (5) jeweils bei Überschreiten des oberen Schwellwertes (So) und bei Unterschreiten des unteren Schwellwertes (Su) ausgebildet ist.

2. Drucküberwachungsvorrichtung nach Anspruch 1, dadurch gekennzeichnet, daß durch den Fensterkomparator (1) eine feste Fensterbreite vorgebbar ist, deren Lage durch einen Sollwertgeber (2) wählbar ist.

3. Drucküberwachungsvorrichtung nach Anspruch 1, dadurch gekennzeichnet, daß die Fensterbreite des Fensterkomparators (1) durch jeweils einen Sollwertgeber (2) für den unteren Schwellwert (Su) und einen Sollwertgeber (3) für den oberen Schwellwert (So) bestimmbar ist.